# EUROPEAN PATENT SPECIFICATION

(11) **EP 3 454 851 B1**
(45) Date of publication and mention of the grant of the patent: **21.10.2026**
(21) Application number: 17795677.8
(22) Date of filing: 08.05.2017
(51) Int. Cl.: A61K 31/4375, A61K 31/5517, A61K 31/551, A61K 31/5513, A61K 31/135, A61K 31/381, A61K 31/485, A61K 31/451, A61K 31/131, A61K 45/06, A61K 9/00, A61P 23/00, A61P 25/20

(54) **COMPOSITION COMPRISING A COMBINATION OF THE BENZOFUROQUINOLIZINE MK-467, AN ALPHA-2-ADRENOCEPTOR AGONIST, AN OPIOID AND A BENZODIAZEPINE FOR USE IN SEDATION AND/OR ANESTHESIA**
ZUSAMMENSETZUNG MIT EINER KOMBINATION AUS DEM BENZOFUROCHINOLIZIN MK-476, EINEM ALPHA-2-ADRENOZEPTORAGONIST, EINEM OPIOID UND EINEM BENZODIAZEPIN ZUR VERWENDUNG IN DER BEHANDLUNG VON SEDIERUNG UND/ODER ANÄSTHESIE
COMPOSITION COMPRENANT UNE COMBINAISON DE LA BENZOFUROQUINOLIZINE MK-467, UN AGONISTE DES RÉCEPTEURS ADRÉNERGIQUES ALPHA-2, UN OPIOÏDE ET UNE BENZODIAZÉPINE À UTILISER EN SÉDATION ET/OU EN ANESTHÉSIE

(30) Priority: 09.05.2016 FI 20165395
(43) Date of publication of application: 20.03.2019
(73) Proprietor: Vetcare Oy, 24101 Salo (FI)
(72) Inventor: VAINIO, Outi, 20100 Turku (FI); RAEKALLIO, Marja, 00280 Helsinki (FI); HONKAVAARA, Juhana, 00530 Helsinki (FI); KALLIO-KUJALA, Ira, 01300 Vantaa (FI); TURUNEN, Heta, 13210 Hämeenlinna (FI); BENNETT, Rachel, Winchester Hampshire SO23 7JB (GB); ADAM, Magdy, 01450 Vantaa (FI)
(74) Representative: Boco IP Oy Ab
(86) International application number: PCT/FI2017/050356
(87) International publication number: WO 2017/194835

(56) References cited:
- EP-A2- 2 612 670
- FR-A1- 2 787 713
- NICOLE G ROLFE ET AL: "Cardiopulmonary and sedative effects of the peripheral [alpha]2-adrenoceptor antagonist MK 0467 administered intravenously or intramuscularly concurrently with medetomidine in dogs", AJVR, VOL. 73, N.5, 1 May 2012 (2012-05-01), pages 587 - 594, XP055440683, Retrieved from the Internet <URL:https://avmajournals.avma.org/doi/pdf/10.2460/ajvr.73.5.587> [retrieved on 20180115]
- DATABASE MEDLINE [online] US NATIONAL LIBRARY OF MEDICINE (NLM), BETHESDA, MD, US; March 2012 (2012-03-01), HONKAVAARA JUHANA ET AL: "Influence of MK-467, a peripherally acting [alpha]2-adrenoceptor antagonist on the disposition of intravenous dexmedetomidine in dogs.", XP002795788, Database accession no. NLM22112381
- DRUG METABOLISM AND DISPOSITION: THE BIOLOGICAL FATE OF CHEMICALS MAR 2012, vol. 40, no. 3, March 2012 (2012-03-01), pages 445 - 449, ISSN: 1521-009X
- ADAM MAGDY ET AL: "Effects of the peripherally acting [alpha]2-adrenoceptor antagonist MK-467 on cardiopulmonary function in sheep sedated by intramuscular administration of medetomidine and ketamine and reversed by intramuscular administration of atipamezole", AMERICAN JOURNAL OF VETERINARY RESEARCH, vol. 79, no. 9, 1 September 2018 (2018-09-01), pages 921 - 932, XP093019536
- SALLA, K. ET AL.: "The cardiopulmonary effects of a peripheral alpha-2- adrenoceptor antagonist, MK -467, in dogs sedated with a combination of of medetomidine and butorphanol", VETERINARY ANAESTHESIA AND ANALGESIA, vol. 41, no. 6, 2014, pages 567 - 574, XP055441279
- VAINIONPÄÄ, M. ET AL.: "Thermographic imaging of superficial temperature in dogs sedated with medetomidine and butorphanol with and without MK-467 (L-659'066)", VETERINARY ANAESTHESIA AND ANALGESIA, vol. 40, no. 2, 2013, pages 142 - 148, XP055441282
- PAKKANEN, S.A.E ET AL.: "Detomidine and the combination of detomidine and MK -467, a peripheral aplha-2 adrenoceptor antagonist, as premedication in horses anaesthetized with isoflurane", VETERINARY ANAESTHESIA AND ANALGESIA, vol. 42, no. 5, 2015, pages 527 - 536, XP055551891, ISSN: 1467-2987, DOI: 10.1111/vaa.12238

## Description

### FIELD OF THE INVENTION

The present invention relates to compositions comprising a substituted benzofuroquinolizine known as MK-467, an α₂-adrenoceptor agonist, an opioid, and a benzodiazepine for use in sedation and/or anesthesia. The invention also relates to MK-467 for use in facilitating onset of sedation and/or anesthesia by facilitating the absorption and distribution of psychoactive drugs.

### BACKGROUND OF THE INVENTION

Psychoactive drugs, particularly opioids, benzodiazepines and NMDA receptor antagonists, such as ketamine are drugs, which are used in the medicine, particularly in the veterinary medicine in analgesia and sedation of animals, as well as in connection with anesthesia for improving the quality of anesthesia. Opioids typically used in animals are fentanyl, morphine, oxymorphine, buprenorphine and butorphanol tartrate. Diazepam, zolazepam, and midazolam are widely used benzodiazepines in veterinary use. Ketamine, tiletamine, dextromethorphan, phencyclidine and methoxetamine are examples of NMDA receptor antagonists, ketamine being commonly used particularly in the veterinary medicine. Opioids, benzodiazepines and NMDA receptor antagonists, such as ketamine may be used alone or in combination with each other.

Opioids act by activating opioid receptors (morphine receptors) particularly at the dorsal horn of the spinal cord. As a result of the activation, mediation of the pain to brains is reduced and the sense of pain is attenuated. Opioids are primarily used for analgesia, including anesthesia.

Benzodiazepines reduce fear and anxiety, and they have muscle relaxing effects on striated muscles. Benzodiazepines enhance the effect of the neurotransmitter gamma-aminobutyric acid (GABA) at the GABA_{A} receptor complexes, where they bind to own benzodiazepine receptors and intensify the effect of GABA in brains, resulting in sedative, hypnotic (sleep-inducing), anxiolytic, anticonvulsant, and muscle relaxant properties. GABA is a mediating substance, retarding the activity of nervous cells in the brain. Thus, benzodiazepines cause general slowdown of nervous pathways and reduction of anxiety.

Ketamine is classified as an NMDA receptor antagonist, but it also acts on opioid receptors and monoamine transporters among others. Ketamine is mainly used for starting and maintaining anesthesia, but also in pain management and as a sedative.

Experimental compound MK-467 (known also as L-659,066) has been suggested for IV administration to attenuate the peripheral vascular effects of α₂-adrenergic agonists. MK-467 passes the blood brain barrier poorly or not at all and its effects are directed solely to peripheral α₂-adrenergic receptors outside the central nervous system.

Effects of MK-467 on the hemodynamic changes induced by medetomidine administration in conscious dogs were studied in Enouri S et al, AJVR, Vol 69, No 6, (2008) 728-736. 0.2 mg/kg of MK-467 was administrated IV as pretreatment, and ten minutes after the administration the dogs received 10 µg/kg dose of medetomidine. Premedication with MK-467 prior to sedation with medetomidine reduced negative cardiovascular alterations induced by medetomidine administration in dogs.

Pagel P et al, J. Cardiothor. Vasc. Anaest., Vol 12, No 4, (1998) 429-434, describes premedication (IV) of dogs with 0.1, 0.2 and 0.4 mg/kg of MK-467, followed after 30 min by administration (IV) of 5 µg/kg dose of dexmedetomidine. MK-467 dose-dependently induced reduction in systemic vascular resistance coupled with an increase in heart rate and cardiac output, resulting in stable mean arterial pressures.

The effects of three doses of MK-467 (250, 500 and 750 µg/kg) in combination of 10 µg/kg of dexmedetomidine (IV) on bispectral index (BIS) and clinical sedation in dogs were evaluated in Restitutti F et al, Vet. Anaest. Analg. Vol 38, (2011) 415-422. BIS is used for monitoring sedation and loss of consciousness during anesthesia. Dexmedetomidine decreased BIS-values and MK-467 increased BIS-values especially with higher doses.

Influence of different doses of MK-467 on cardiovascular effects of dexmedetomidine in dogs were evaluated in Honkavaara J et al, J Vet. Pharmacol. Therap. (2011) 38, 332-337. Dexmedetomidine 10 µg/kg with 250, 500 or 750 µg/kg and MK-467 (IV) were used. MK-467 dose-dependently attenuated dexmedetomidine induced increase in systemic vascular resistance and blood pressure and the consequent reductions in heart rate and cardiac index.

Cardiopulmonary effects of MK-467 in dogs sedated with medetomidine and butorphanol via IV and IM administration was studied in Salla K et al, Vet. Anaest. Analg. (2014)41, 567-574. MK-467 attenuated the cardiovascular effects of medetomidine-butorphanol combination after IV and IM administration.

Pakkanen, S A E et al. Vet. Anaest. Analg. (2015)42, 527-536 describes concurrent IV administration of detomidine (20 µg/kg) and MK-467 (200 µg/kg) as premedication in horses anaesthetisized 10 minutes later with ketamine (2.2 mg/kg) and midazolam (0.06 mg/kg) administered IV in the same syringe.

α₂-adrenergic receptors are located on pre-junctional terminals in the central nervous system where they inhibit the release of norepinephrine in the form of negative feedback. They are further located post-synaptically on the smooth vascular muscle cells of certain blood vessels, such as those found in arterioles or on veins. The α₂-adrenergic receptors bind both norepinephrine released by the sympathetic postganglionic fibers and epinephrine released by the adrenal medulla.

Common effects of α₂-adrenergic receptors include suppression of release of norepinephrine by negative feedback, transient hypertension followed by sustained hypotension, decrease in heart rate, vasoconstriction of certain arteries, venoconstriction of some veins, as well as decrease of the motility of the GI track by relaxing its smooth muscles, and sedation and analgesia.

Agonists (activators) of the α₂-adrenergic receptors are frequently used as sedatives and in anesthesia where they affect sedation, muscle relaxation and analgesia through effects on the central nervous system (CNS).

Substituted imidazoles and substituted thiazines are α₂-adrenergic receptor agonists used in veterinary medicine in sedation, analgesia and premedication, and in humans for similar purposes in intensive care. The activation of α₂-adrenergic receptors located outside the CNS, such as postsynaptically located receptors on the vascular smooth muscle cells, induces vasoconstriction resulting in hypertension and significantly reduced heart rate, whereby the oxygen delivery to tissues is reduced.

The effect of substituted imidazoles and substituted thiazines is based on the activation of presynaptic α₂-adrenergic receptors located in brains, which causes sedation, analgesia, and decrease of level of consciousness and fear.

Clonidine, romifidine, medetomidine, dexmedetomidine and detomidine are examples of such substituted imidazoles; xylazine is an example of substituted thiazines, useful as α₂-adrenergic agonists.

Medetomidine, a racemic mixture of dexmedetomidine and levomedetomidine, is a popular sedative and pre-anesthetic drug used in small animal practice. Medetomidine administration is associated with significant alterations in cardiovascular functions, such as dramatic increase in arterial blood pressure, in systemic and pulmonary vascular resistance, and in myocardial workload after IV administration. Further, reduction in heart rate and in cardiac output decrease global Do₂ (oxygen delivery) by at least 50%. Further adverse effects, such as vasoconstriction, bradycardia and decreased respiratory rate are typically caused by medetomidine. In some cases the level and quality of sedation and analgesia may not be sufficient and thus medetomidine may be combined with opioids, such as butorphanol, which has pure κ-agonist, partial µ-agonist and δ-agonist properties, with benzodiazepines, such as midazolam, or with NMDA receptor antagonists, such as ketamine.

Dexmedetomidine, the pharmacologically active isomer of medetomidine, has similar side effects to medetomidine, such as vasoconstriction, acute α₂-adrenoceptor agonist induced bradyarrhythmias and decreased respiratory rate. In animals, such as dogs, both dexmedetomidine and medetomidine produce dose dependent degree of sedation, higher doses will prolong the sedative effects.

Detomidine is a α₂-adrenergic agonist producing dose-dependent sedative and analgesic effects. Due to inhibition of the sympathetic nervous system, it also has cardiac and respiratory effects and an antidiuretic action. For example, after administration to a horse, a short period of reduced coordination is characteristically followed by immobility and a firm stance with front legs spread. Following administration there is an initial increase in blood pressure, followed by sinus bradycardia, sinoatrial blocks and second-degree atrioventricular blocks. The horse commonly sweats to excess, especially on the flanks and the neck.

Detomidine is typically used for sedation and anesthetic premedication in horses and other large animals, commonly combined with butorphanol, midazolam or diazepam for increased analgesia and depth of sedation. It may also be used in conjunction with ketamine for intravenous anesthesia of short duration.

Several approaches have been studied to enhance and facilitate the onset of sedation and/or anesthesia while simultaneously minimizing the adverse effects, such as those of α₂-adrenergic agonists, including the dose-dependency of the cardiovascular alterations and the effects of co-administration of anticholinergic agents.

Intravenous administration of sedative drugs may be a challenge to fractious or uncooperative animals. Thus, in many cases it is preferable to administer sedatives intramuscularly.

Medetomidine is used as sedative or pre-medicament in small animal medicine, and it can be administered intravenously, intramuscularly, and subcutaneously (SC). When compared to IV administration, in IM or SC administration of medetomidine it typically takes more time before the sedation of the animal is sufficient for starting the procedure, operation etc. due to the need of absorption of active ingredients to circulation. It takes more of the veterinary's time and less patients can be treated daily. The problem is similar with other substituted imidazoles and substituted thiazines.

Rolfe et al, AJVR, 73(5), (2012) 587-94, describes the use 20 µg/kg of medetomidine, IM, alone or concurrently with MK-467 (0.4 mg/kg, IM), and 10 µg of medetomidine/kg, IV, alone or concurrently with MK-467 (0.2 mg/kg, IV), in dogs in a randomized crossover study. Concurrent administration was carried out in separate syringes at different locations. Heart rate (HR), mixed-venous partial pressure of oxygen (Pvo(2)), and cardiac index (CI) were significantly lower and mean arterial blood pressure (MAP), systemic vascular resistance (SVR), and oxygen extraction ratio (ER) were significantly higher after administration of medetomidine IM or IV, compared with baseline values. Administration of medetomidine and MK-467 IM caused a significantly higher heart rate, CI, and Pvo(2) and significantly lower MAP, SVR, and ER for 60 to 90 minutes than did IM administration of medetomidine alone. Administration of medetomidine and MK-467 IV caused a significantly higher CI and Pvo(2) and significantly lower MAP, SVR, and ER for 45 to 90 minutes than did IV administration of medetomidine alone. No significant difference in sedation was noticed.

Parenteral extravascular administration is commonly used in administration of psychoactive drugs, such as sedatives and anesthetic drugs in animals. However, it typically takes more time before the full sedative and/or anesthetic effect is reached than after IV route, which also increases the total time needed for carrying out the desired operation or procedure. Further, it also takes more time before the subject is recovered from the sedation or anesthesia.

### SUMMARY OF THE INVENTION

An object of the invention is to provide a composition for use in sedation and/or anesthesia, wherein the composition comprises a combination of MK-467, an α₂-adrenoceptor agonist, an opioid, and a benzodiazepine, wherein the product is administered locally at the same time and to the same site of administration using parenteral extravascular administration to a subject in need of sedation and/or anesthesia.

Another object of the invention is to provide MK-467 for use in facilitating onset of sedation and/or anesthesia, wherein a composition comprising a combination of MK-467, an a2-adrenoceptor agonist, an opioid, and a benzodiazepine is administered locally at the same time and to the same site of administration using parenteral extravascular administration to a subject in need of sedation and/or anesthesia.

Particularly the present invention relates to compositions comprising MK-467, an α₂-adrenoceptor agonist, and a psychoactive drug selected from opioids, benzodiazepines, and combinations thereof, for use in sedation and/or anesthesia using parenteral extravascular administration to a subject in need of sedation and/or anesthesia, whereby the onset of sedation and/or anesthesia is enhanced and/or facilitated.

The present disclosure also relates to a method for sedation and/or anesthesia, where a composition comprising MK-467 and a psychoactive drug selected from opioids, benzodiazepines, NMDA receptor antagonists and combinations thereof, is administered to a subject in need of sedation and/or anesthesia, using parenteral extravascular administration.

Characteristic features of the invention are presented in the appended claims.

### DEFINITIONS

The term "anesthesia" refers here to an induced, temporary state with one or more of the following characteristics: analgesia (relief from or prevention of pain), immobility, paralysis (extreme muscle relaxation), amnesia (loss of memory), and unconsciousness.

The term "sedation" refers here to calming through effects on the central nervous system (CNS), and it includes here tranquillization, immobilization, muscle relaxation and analgesia.

The term "α₂-adrenoceptor agonists" refers here to α₂-adrenergic receptor agonists, which are used for affecting sedation. The term "psychoactive drug" means here opioids, benzodiazepines, NMDA receptor antagonists and combinations thereof, which are drugs used in the medicine in anesthesia, analgesia and sedation of mammals and vertebrates in general.

The term "NMDA receptor antagonist" refers here to a class of anesthetics that work to antagonize or inhibit the action of the N-Methyl-D-aspartate receptor (NMDAR). They are used as anesthetics for animals and for humans; the state of anesthesia they induce is referred to as dissociative anesthesia.

The term "α₂-adrenoceptos antagonist" refers here to compounds useful in the prevention and/or reversing the effects of α₂-adrenoceptor agonists.

The expression "parenteral extravascular administration" refers here to administration by the intramuscular route (IM), subcutaneous route (SC), transdermal route, transmucosal route, and intraperitoneal route (IP).

The expression "subject" refers here to humans and animals.

The expression "intramuscular route" refers here to administration of the substance into a muscle, typically using injections.

The expression "subcutaneous route" refers here to administration of the substance into the subcutis, which is the layer of skin directly below the dermis and epidermis, typically using injections.

The expression "transdermal route" refers here to administration of a substance by diffusion through the intact skin, typically using transdermal patches, gels, sprays etc.

The expression "transmucosal route" refers here to administration of the substance by diffusion through a mucous membrane, typically as sublingual, sublabial, buccal, rectal, pulmonary, or intravaginal preparations.

The expression "intraperitoneal route" refers here to administration of the substance into a coelom, such as abdomen, typically using injections.

### BRIEF DESCRIPTION OF THE DRAWINGS

Figure 1 presents plasma butorphanol concentrations with and without MK-467, in the presence of medetomidine and midazolam, mixed in the same syringe.
Figure 2 presents plasma midazolam concentrations with and without MK-467, in the presence of medetomidine and butorphanol, mixed in the same syringe.
Figure 3 presents the visual sedation scores of compositions with and without MK-467.
Figure 4 presents the descriptive sedation scores of compositions with and without MK-467.

### DETAILED DESCRIPTION OF THE INVENTION

It was surprisingly found that using parenteral extravascular administration of a composition comprising MK-467, an α2-adrenoceptor agonist, an opioid, and a benzodiazepine at the same time and to the same site of administration to a subject in need of sedation and/or anesthesia, the absorption rate and the speed of distribution of the psychoactive drug is increased. With the invention the development of sedative and/or anesthetic effect of the psychoactive drug was accelerated significantly, particularly that of an opioid even three-fold and that of a benzodiazepine even four-fold when compared with administration without MK-467.

The "treatment" refers to sedation and/or anesthesia, and it includes tranquillization, immobilization, muscle relaxation, analgesia, and premedication.

The active compounds (MK-467, α₂-adrenoceptor agonist, opioid and benzodiazepine) are administered using parenteral extravascular administration in one composition, locally, at the same time and to the same site of administration, whereby surprisingly fast absorption and distribution of the psychoactive drug and the α₂-adrenoceptor agonist are achieved. MK-467 is effective after parenteral extravascular administration, when combined to a psychoactive drug, and with the α₂-adrenoceptor agonist.

The psychoactive drug is used for treating a mammal species for sedation and/or anesthesia, including tranquillization, immobilization, analgesia, and premedication. MK-467 acts as α₂-adrenoceptor antagonist on post-synaptically located receptors on the vascular smooth muscle cells. The onset of sedation and/or anesthesia is enhanced or accelerated significantly.

The composition is administered using parenteral extravascular administration, which is selected from administration by the intramuscular route (IM), intraperitoneal route (IP), subcutaneous route (SC), transdermal route, and transmucosal route.

According to the present invention the composition comprises MK-467, psychoactive drug and α₂-adrenoceptor agonist. The adverse effects of α₂-adrenoceptor agonists can be reduced or even avoided after parenteral extravascular administration of the composition to a subject. The vasodilatation effect of MK-467 is particularly pronounced when the composition comprises a α₂-adrenoceptor agonist causing vasoconstriction. MK-467 has also vasodilating effect when the veins are constricted because of physiological or pathophysiological reasons.

According to the present invention the composition comprises MK-467, psychoactive drug and α₂-adrenoceptor agonist, where the psychoactive drug comprises an opioid and a benzodiazepine.

According to one preferable embodiment the composition comprises MK-467, psychoactive drug and α₂-adrenoceptor agonist, where the psychoactive drug comprises an opioid, a benzodiazepine and a NMDA receptor antagonist.

MK-467 increases the absorption and distribution of the opioid, the benzodiazepine and the NMDA receptor antagonist, particularly when the composition also comprises the α₂-adrenoceptor agonist. This can also be seen in the examples where the liquid composition comprising MK-467, butorphanol (opioid), midazolam (benzodiazepine) and medetomidine (α₂-adrenoceptor agonist) was administered to dogs using parenteral extravascular administration. Maximum plasma concentration of butorphanol was reached after 10 min, whereas without MK-467 it took 30 min, and respective values for midazolam were 5 min and 20 min.

It was further surprising that after parenteral extravascular administration of the composition, particularly the composition comprising MK-467, an opioid, a benzodiazepine and α₂-adrenoceptor agonist, to a subject in need of treatment, particularly sedation and/or anesthesia, in addition to the increased absorption rate and speed of distribution of the opioid and benzodiazepine, the maximum plasma concentration of the opioid is increased 35 % and the maximum plasma concentration of the benzodiazepine 57 %.

Particularly, in veterinary medicine, the parenteral extravascular route of administration is the most commonly used and preferred route when administering analgesics and sedatives.

By the composition of the invention the full effect of the psychoactive drugs is reached more rapidly, and the onset of sedation and/or anesthesia is enhanced or accelerated significantly. This can be seen also when the composition comprises additionally an α₂-adrenoceptor agonist.

A composition providing rapidly the desired effect on the subject, such as an animal, has an advantageous effect on the well-being of the subject, such as an animal, because the total time needed for an operation etc. is reduced, and the practitioner, such as the veterinarian, can treat more patients. The treated patient, such as the animal, can be released from clinic earlier because of the shortened treatment time.

MK-467 is also effective when used with other α₂-adrenoceptor antagonists which are used to prevent and/or reverse the effects of any alpha-2 adrenoceptor agonists in a subject, such as an animal. It was surprisingly found that the effects of alpha-2 adrenoceptor agonists (induced by the composition of the invention) can be rapidly reversed, after an operation or procedure is finished or it is desirable for another reason to reverse the effects, by administering the α₂-adrenoceptor antagonists to the subject, preferably by IM administration.

The present invention is directed to a composition comprising MK-467, an α₂-adrenoceptor agonist, opioid, benzodiazepine, for use in anesthesia and/or sedation, where the composition is administered locally at the same time and to the same site of administration using parenteral extravascular administration to a subject in need of anesthesia and/or sedation, whereby the onset of sedation and/or anesthesia is enhanced and/or facilitated.

The disclosure also relates to MK-467 for use in facilitating onset of sedation and/or anesthesia, where a composition comprising MK-467, an α₂-adrenoceptor agonist, opioid, and benzodiazepine, is administered locally at the same time and to the same site of administration to a subject in need of sedation and/or anesthesia using parenteral extravascular administration.

Sedation, particularly induced by the α₂-adrenoceptor agonist can be conveniently reversed by administering α₂-adrenoceptor antagonist to the subject.

According to another embodiment, the present disclosure is directed to a composition comprising MK-467 and a psychoactive drug and α₂-adrenoceptor agonist for use in anesthesia and/or sedation, where the composition is administered using parenteral extravascular administration to a subject in need of anesthesia and/or sedation, and a α₂-adrenoceptor antagonist is administered to the subject for use in reversing the anesthesia and/or sedation. The composition of the invention is administered to a subject to provide a prescribed or approved dosage of the psychoactive drug. The dosage of the psychoactive drug depends on the compound which is used and on the subject which receives treatment. Any doses of the psychoactive drug which are used to treat humans, or any domestic or wild animal species are suitable.

The composition of the invention is administered to a subject to provide a prescribed or approved dosage of the α₂-adrenoceptor agonist. The dosage of the α₂-adrenoceptor agonist depends on the compound which is used and on the subject which receives treatment. Any doses of the α₂-adrenoceptor agonist which are used to treat humans, or any domestic or wild animal species are suitable.

The subject is selected from humans and animals. The animals are understood to mean vertebrate animal species selected from domestic animals and wild animals, including mammals, fish, birds, and reptiles. Examples of said animals are wild animals, animals kept in parks and zoos, laboratory animals, pets and livestock. The domestic animals include dogs, cats, rodents, reptiles, birds and other pets, horses, donkeys, pigs, and ruminants including bovine animals, sheep and goats, poultry, fish etc.

### MK-467

MK-467 refers here to a peripherally acting α₂-adrenoceptor antagonist, known also by code as L-659,066. MK-467 has the following systematic chemical name N-[2-[(2R,12bS)-2'-oxospiro[1,3,4,6,7,12b-hexahydro-[1]benzofuro[2,3-a]quinolizine-2,5'-imidazolidine]-1'-yl]-ethyl]-methanesulfonamide (IUPAC). It is a spirocyclic substituted benzofuroquinolizine having the chemical formula I:

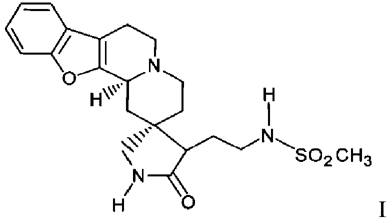

The composition of the invention is administered to a subject to provide a dosage of 1-5000 µg/kg of MK-467, preferably 10-3000 µg/kg of MK-467, particularly preferably 50-1500 µg/kg of MK-467.

### Psychoactive drug

The psychoactive drug is selected from opioids, benzodiazepines, NMDA receptor antagonists and any combinations thereof.

The opioid is selected from fentanyl, morphine, oxymorphine, buprenorphine, butorphanol tartrate, methadone, levomethadone, alfentanil, sufentanil, remifentanil, nalbuphine, pentazocine, oxymorphone, hydromorphone, preferably from fentanyl, morphine, oxymorphine, buprenorphine and butorphanol tartrate.

The benzodiazepine is selected from midazolam, zolazepam, and diazepam.

The NMDA receptor antagonist is selected from ketamine, tiletamine, dextromethorphan, phencyclidine and methoxetamine, preferably ketamine.

The dosage of the psychoactive drug is suitably in the range of 1-5000 µg/kg.

The dosage of fentanyl is suitably 1-10 µg/kg.

The dosage of morphine is suitably 100-2000 µg/kg.

The dosage of oxymorphine is suitably 10-2000 µg/kg.

The dosage of buprenorphine is suitably 5-100 µg/kg.

The dosage of butorphanol tartrate is suitably 10-1000 µg/kg.

The dosage of midazolam is suitably 50-5000 µg/kg.

The dosage of diazepam is suitably 50-500 µg/kg.

The dosage of zolazepam is suitably 100-20000 µg/kg.

The dosage of ketamine is suitably 500-20000 µg/kg.

The dosage of tiletamine is suitably100-20000 µg/kg.

### α₂-adrenoceptor agonist

The α₂-adrenoceptor agonist is selected from peripherally acting α₂-adrenoceptor agonists. Suitable α₂-adrenoceptor agonists are substituted imidazoles and substituted thiazines. Preferably the α₂-adrenoceptor agonist is medetomidine ((RS)-4-[1-(2,3-dimethylphenyl)ethyl]-3H-imidazole of formula II), dexmedetomidine ((S)-4-[1-(2,3-Dimethylphenyl)ethyl]-3H-imidazole of formula III), detomidine (4-[(2,3-dimethylphenyl)methyl]-3H-imidazole of formula IV), romifidine (N(2-bromo-6-fluorophenyl)-4,5-dihydro-1H-imidazol-2-amine of formula V, clonidine (N-(2,6-dichlorophenyl)-4,5-dihydro-1H-imidazol-2-amine of formula VI) or xylazine (N-(2,6-dimethylphenyl)-5,6-dihydro-4H-1,3-thiazin-2-amine of formula VII), which are all structurally similar to each other. Particularly preferably the α₂-adrenoceptor agonist is detomidine, medetomidine, dexmedetomidine, romifidine or xylazine. Also, any pharmaceutically acceptable salts of said compounds may be used in the invention.

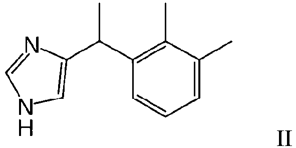

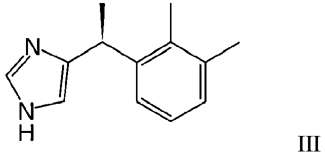

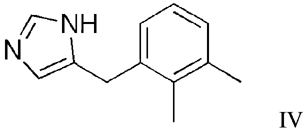

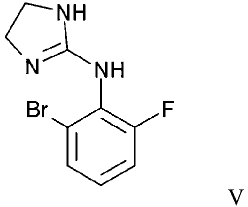

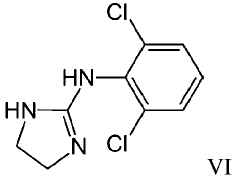

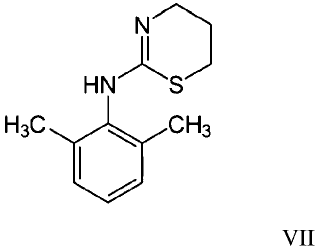

The dosage of the α₂-adrenoceptor agonist is suitably in the range of 0.05-20000 µg/kg.

The dosage of detomidine is suitably 0.1-5000 µg/kg, preferably 0.2-3000 µg/kg, particularly preferably 0.5-1000 µg/kg.

The dosage of medetomidine is suitably 0.1-5000 µg/kg, preferably 0.2-3000 µg/kg, particularly preferably 0.5-1000 µg/kg.

The dosage of dexmedetomidine is suitably 0.05-3000 µg/kg, preferably 0.1-2000 µg/kg, particularly preferably 0.1-1000 µg/kg.

The dosage of romifidine is suitably 0.1-5000 µg/kg, preferably 0.2-3000 µg/kg, particularly preferably 0.5-1500 µg/kg.

The dosage of clonidine is suitably 0.1-5000 µg/kg, preferably 0.2-3000 µg/kg, particularly preferably 0.5-1000 µg/kg.

The dosage of xylazine is suitably 1-20000 µg/kg, preferably 10-10000 µg/kg, particularly preferably 50-5000 µg/kg.

According to the invention the compounds MK-467, the psychoactive drug and the α₂-adrenoceptor agonist are administered at the same time, in the same composition, to the same administration site of the subject, using parenteral extravascular administration.

Preferably the administration of the composition is carried out by the intramuscular route (IM), intraperitoneal (IP), subcutaneous route (SC), transdermal route, or transmucosal route.

### Composition

Examples of the compositions of the invention, for parenteral extravascular administration, comprise solutions for intramuscular use, intraperitoneal and subcutaneous use (IM, IP and SC solutions); gels, sprays, ointments, creams and patches for transdermal use (transdermal gels, sprays, ointments, creams and patches); and gels, ointments, creams, sprays and suppositories for transmucosal use (transmucosal gels, ointments, creams, sprays and suppositories). Transmucosal use includes here also sublingual and intravaginal administration.

Solutions for intramuscular (IM), intraperitoneal (IP) and subcutaneous (SC) use (IM, IP or SC composition) may comprise saline or another physiologically acceptable aqueous medium, where MK-467, α₂-adrenoceptor agonist, opioid and benzodiazepine are dissolved to desired concentrations. Pharmaceutically acceptable excipients, such as solubility and stability enhancing agents and preservatives, known in the art may be added if necessary to the composition.

For example, the IM, IP, or SC composition may comprise 0.1-500 mg/ml of MK-467, 0.1-500 mg/ml of the psychoactive drug and 0.01-500 mg/ml of the α₂-adrenoceptor agonist and a suitable preservative. Sterile solutions for intramuscular (IM) and subcutaneous use may be packed in any vials, bottles, syringes etc. devices or containers known in the art.

Compositions for transdermal use may comprise one or more pharmaceutically acceptable carriers, supports, MK-467, the psychoactive drug and the α₂-adrenoceptor agonist in dissolved state in a pharmaceutically acceptable medium, and excipients, including preservatives known in the art, depending on whether the composition is in the form of a gel, spray, cream, ointment or patch etc.

For example, the transdermal composition may comprise 0.1-1000 mg/ml of MK-467, 0.1-500 mg/ml of the psychoactive drug and 0.01-500 mg/ml of the α₂-adrenoceptor agonist.

Compositions for transmucosal use may comprise one or more pharmaceutically acceptable carriers, MK-467, the psychoactive drug and the α₂-adrenoceptor agonist in dissolved state in a pharmaceutically acceptable medium, and excipients including preservatives known in the art, depending on whether the composition is in the form of a gel, spray, ointment or cream etc.

For example, a transmucosal composition may comprise 0.1-1000 mg/ml of MK-467, 0.1-500 mg/ml of the psychoactive drug and 0.01-100 mg/ml of the α₂-adrenoceptor agonist.

For example, an oromucosal gel for sublingual use may comprise 0.1-1000 mg/ml of MK-467, 0.1-500 mg/ml of the psychoactive drug and 0.01-100 mg/ml of the α₂-adrenoceptor agonist, dissolved in an aqueous carrier, optionally with solubility enhancing agent(s) and surfactant(s), where said composition is formed to a gel with a gel forming agent, such as hydroxypropyl cellulose or the like.

### Reversing of effects of α₂-adrenoceptor agonist

As a further advantage of the invention, when a α₂-adrenoceptor antagonist, other than MK-467, was used for reversing the central and peripheral effects of the α₂-adrenoceptor agonist after administration of the composition of the invention to a subject, such as an animal, the recovery of the subject takes place more smoothly and rapidly because MK-467 also enhances distribution and absorption of said α₂-adrenoceptor antagonists, which in turn facilitates the elimination of the α₂-adrenoceptor agonist.

α₂-adrenoceptor antagonists, other than MK-467, may be used for preventing and/or reversing the effects of α₂-adrenoceptor agonists. α₂-adrenoceptor antagonists useful for reversing effects of substituted imidazoles and substituted thiazines are selected from a group consisting of idazoxan, tolazoline, yohimbine, rauwolskine, atipamezole, mirtazapine and (±)-2-methyl-1,2,3,4,10,14b-hexahydropyrazino[2,1-a]pyrido[2,3-c][2]benzazepine. In a preferable embodiment, atipamezole is used.

Said α₂-adrenoceptor antagonists are administered using parenteral extravascular administration, suitably using IM, IP or SC administration. The dosage of the α₂-adrenoceptor antagonists is generally from 10 to 1000 µg/kg, depending on the subject and the α₂-adrenoceptor agonist used. Preferably IM administration is used.

Atipamezole is a specific α₂-adrenoceptor antagonist, which is used commonly for reversing the central and peripheral effects of medetomidine and dexmedetomidine in animals.

The present invention provides several advantages for the subject and for the practitioner. The inventors have found that after parenteral extravascular administration of the composition comprising MK-467, the psychoactive drug and the α₂-adrenoceptor agonist, to a subject, such as an animal, in need of sedation and/or anesthesia, the absorption rate and the speed of distribution of the psychoactive drug and the α₂-adrenoceptor agonist is increased even four-fold when compared with administration of the psychoactive drug and the α₂-adrenoceptor agonist without MK-467. The onset of action of the psychoactive drug and the α₂-adrenoceptor agonist, particularly the onset of sedation and/or anesthesia starts more rapidly as can be seen from the examples. Long waiting periods can be avoided as less time is needed before an operation or procedure can be started.

Further, the effects of the α₂-adrenoceptor agonist are reversed faster due to the improved distribution and absorption of α₂-adrenoceptos antagonists. The subject (the animal) recovers more rapidly and long recovery periods after the operation or procedure can be avoided or at least decreased significantly.

The wellbeing of the patient, such as an animal is improved because of less side-effects, easier administration, faster onset and recovery when sedatives are used and shorter and smoother visits to the practitioner, such as veterinarian. Also potential re-sedation after the reversing α₂-adrenoceptor antagonist, such as atipamezole, induced recovery from sedation is inhibited by the invention and the composition comprising MK-467, the psychoactive drug and the α₂-adrenoceptor agonist.

More patients can be treated with a period of time because long waiting periods can be avoided both in the onset of sedation and recovery, which increases the productivity of the veterinarian and brings economic advantages.

Further, as the onset of the sedation and/or anesthesia is faster, potentially dangerous animals can be sedated without unnecessary delay, which also increases the safety of the veterinarian.

### Examples

The invention will now be illustrated with the following examples and with reference to the drawings.

### Example 1

### Plasma concentrations of butorphanol and midazolam in dogs after IM administration of a composition comprising MK-467, butorphanol, midazolam and medetomidine, compared with composition without MK-467

A liquid aqueous composition comprising MK-467, butorphanol tartrate, midazolam and medetomidine was administered to a dog intramuscularly (IM) to provide a dosage of MK-467 500 µg/kg, butorphanol 100 µg/kg, midazolam 200 µg/kg and medetomidine 20 µg/kg. MK-467 enhanced and improved the absorption and distribution of butorphanol and midazolam, as well as medetomidine.

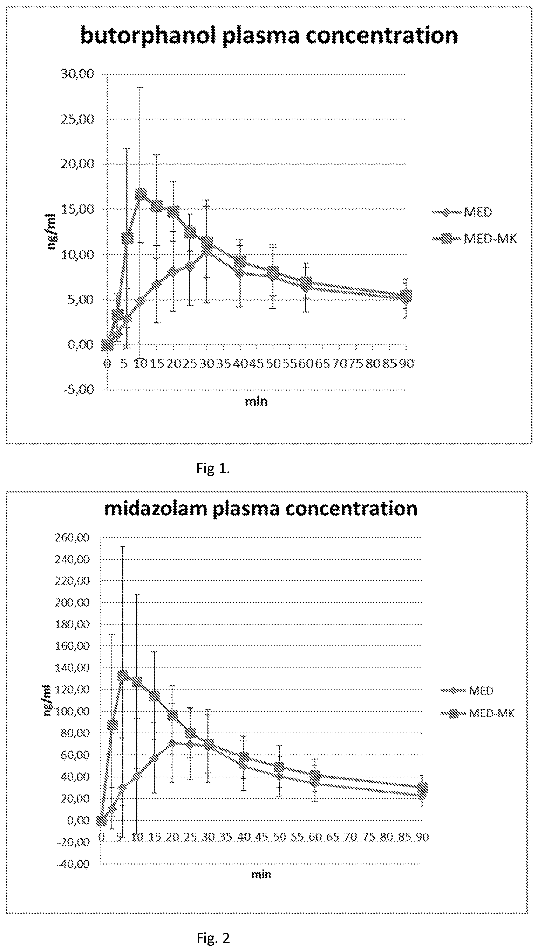

Peak plasma concentration of butorphanol was 17 ng/ml in the presence of MK-467 and 11 ng/ml (200 µg/kg) without MK-467. The butorphanol peak plasma concentration was reached within 10 minutes from the IM administration with MK-467, and within 30 minutes without MK-467, respectively. Butorphanol plasma concentrations are presented graphically in Fig. 1.

Peak plasma concentration of midazolam was 133 ng/ml in the presence of MK-467 and 71 ng/ml without MK-467. The midazolam peak plasma concentration was reached within 5 minutes from the IM administration with MK-467, and within 30 minutes without MK-467. Midazolam plasma concentrations are presented graphically in Fig. 2.

Five purpose-bred, adult beagles were used for this study.

Study design: A prospective, randomized, experimental cross-over study.

Each dog was treated for two times with a 14-day wash-out period:
Treatment 1. medetomidine (20 µg/kg) + butorphanol (100 µg/kg) + midazolam (0.2 mg/kg) Treatment 2. medetomidine (20 µg/kg) + MK-467 (MK) (500 µg/kg) + butorphanol (100 µg/kg) + midazolam (0.2 mg/kg).

For treatment 1. 1 mL of medetomidine (Dorbene 1 mg/mL) was diluted with 1 mL of saline. For treatment 2. 25 mg of MK-467 was mixed with 1 mL of medetomidine, and 1 mL of saline was added. The mixture, butorphanol (Torpudor 10 mg/mL) and midazolam (Midazolam Hameln 5 mg/mL) were aspirated into a single syringe. The end volume of the injectable in both treatments was 0.09 mL/kg.

Dog was positioned laterally and the drugs were injected IM into the epaxial muscle. Central venous blood was sampled into EDTA-tubes at 0, 3, 6, 10, 15, 20, 25, 30, 40, 50, 60 and 90 minutes after drug administration. The plasma concentrations were analyzed with LC/MS/MS for plasma concentrations of dexmedetomidine, midazolam and butorphanol.

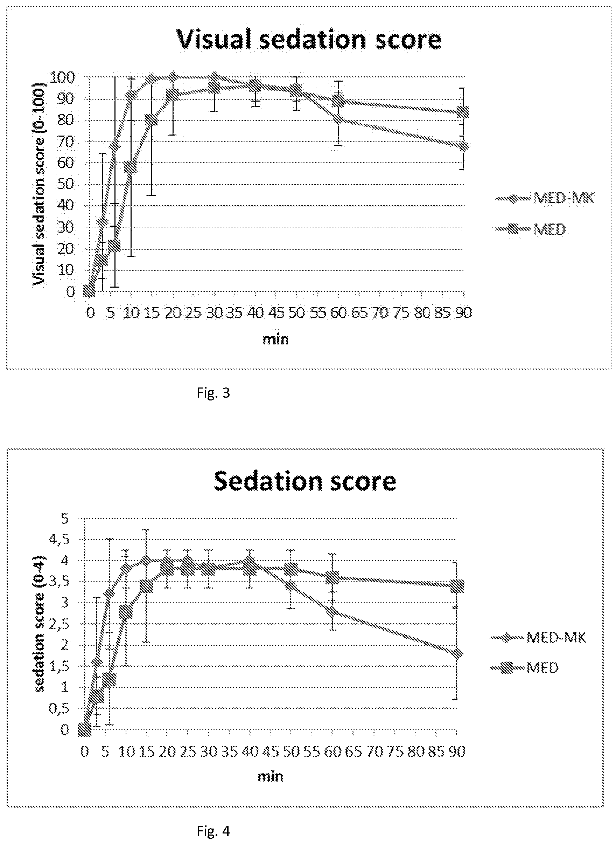

The level of sedation was assessed with both visual sedation score (VSS, analog scale of 0-100) presesnted in Figure 3 and descriptive sedation score (0= no sedation; 1= can stand / walk, but ataxic; 2= recumbent, strong resistance to lateral positioning; 3= recumbent, allows lateral positioning, reacts to hand clap; 4=recumbent, no resistance to lateral positioning, does not react to hand clap) presented in Figure 4. The scores were always assessed by the same, single person (blinded for the treatment) before and at 0, 3, 6, 10, 15, 20, 25, 30, 40, 50, 60 and 90 after the injection.

Both Figures 3 and 4 confirm the faster onset of sedation when the composition contained MK-467.

### Example 2

### Randomized cross-over study in sheep with compositions comprising ketamine, medetomidine and MK-467

### Study design

Each sheep (N=9) will get each of the following treatments with a randomized cross-over design; all drugs mixed in the same syringe and administered intramuscularly (IM) as follows:
- Medetomidine (30 µg/kg) + ketamine (1 mg/kg)
- Medetomidine (30 µg/kg) + ketamine (1 mg/kg) + MK-467 (150 µg/kg)
- Medetomidine (30 µg/kg) + ketamine (1 mg/kg) + MK-467 (300 µg/kg)
- Medetomidine (30 µg/kg) + ketamine (1 mg/kg) + MK-467 (600 µg/kg)

Atipamezole (150 µg/kg IM) will be administered in each treatment at 60 minutes after the initial injection.

Blood will be collected from the carotid artery at 3, 6, 10, 15, 20, 25, 30, 40, 50 and 90 minutes after the first injection, and plasma medetomidine and ketamine concentration will be analyzed with liquid chromatography and tandem mass spectrometry (LC-MS/MS). Maximum plasma concentration of ketamine and medetomidine (Cₘₐₓ) and the time to maximum concentration (Tₘₐₓ) will be calculated from the plasma drug concentration-time data.

The present invention has been described herein with reference to specific embodiments. It is, however clear to those skilled in the art that the invention may be varied within the bounds of the claims.

## Claims

1. A composition for use in sedation and/or anesthesia, **characterized in that** the composition comprises a combination of MK-467, an α₂-adrenoceptor agonist, an opioid, and a benzodiazepine, wherein the composition is administered locally at the same time and to the same site of administration using parenteral extravascular administration to a subject in need of sedation and/or anesthesia.

2. The composition for use according to claim 1, **characterized in that** the opioid is selected from fentanyl, morphine, oxymorphine, buprenorphine, butorphanol tartrate, methadone, levomethadone, alfentanil, sufentanil, remifentanil, nalbuphine, pentazocine, oxymorphone, and hydromorphone, preferably from fentanyl, morphine, oxymorphine, buprenorphine and butorphanol tartrate.

3. The composition for use according to claim 1 or 2, **characterized in that** the benzodiazepine is selected from midazolam, zolazepam, and diazepam.

4. The composition for use according to any one of claims 1-3, **characterized in that** the composition further comprises an NMDA receptor antagonist selected from ketamine, tiletamine, dextromethorphan, phencyclidine and methoxetamine, preferably ketamine.

5. The composition for use according to any one of claims 1-4, **characterized in that** the α₂-adrenoceptor agonist is selected from medetomidine, dexmedetomidine, detomidine, romifidine, clonidine and xylazine.

6. The composition for use according to any one of claims 1-5, **characterized in that** the subject is selected from humans and animals, preferably from wild animals and domestic animals.

7. The composition for use according to claim 6, **characterized in that** the animals are selected from vertebrates, preferably from mammals, fish, birds and reptiles.

8. The composition for use according to any one of claims 1-7, **characterized in that** the composition is administered to a subject to provide a dosage of 1-5000 µg/kg of MK-467 and 1-5000 µg/kg of the psychoactive drug opioid, benzodiazepine and NMDA receptor antagonist.

9. The composition for use according to any one of claims 1-8, **characterized in that** the composition is administered to a subject to provide a dosage of 1-5000 µg/kg of MK-467, 1-5000 µg/kg of the psychoactive drug and 0.05-20000 µg/kg of the α₂-adrenoceptor agonist.

10. The composition for use according to any one of claims 1-9, **characterized in that** the composition is selected from solutions for intramuscular use, intraperitoneal use and subcutaneous use; from gels, sprays, ointments, creams and patches for transdermal use; and from gels, ointments, creams, sprays and suppositories for transmucosal use.

11. The composition for use according to any one of claims 1-10, **characterized in that** the composition is a solution for intramuscular use, intraperitoneal use or subcutaneous use and it comprises 0.1-500 mg/ml of MK-467 and 0.1-500 mg/ml of the psychoactive drug.

12. The composition for use according to any one of claims 1-10, **characterized in that** the composition is a gel, spray, ointment, cream or patch for transdermal use and it comprises 0.1-1000 mg/ml of MK-467 and 0.1-500 mg/ml of the psychoactive drug and 0.01-500 mg/ml of the α₂-adrenoceptor agonist.

13. The composition for use according to any one of claims 1-10, **characterized in that** the composition is a gel, ointment, cream, spray or suppository for transmucosal use and it comprises 0.1-1000 mg/ml of MK-467 and 0.1-500 mg/ml of the psychoactive drug and 0.01-100 mg/ml of the α₂-adrenoceptor agonist.

14. MK-467 for use in facilitating onset of sedation and/or anesthesia, **characterized in that** a composition comprising a combination of MK-467, an α₂-adrenoceptor agonist, an opioid, and a benzodiazepine, is administered locally at the same time and to the same site of administration to a subject in need of anesthesia and/or sedation using parenteral extravascular administration.

15. MK-467 for use in facilitating onset of sedation and/or anesthesia according to claim 14, **characterized in that** the opioid is selected from fentanyl, morphine, oxymorphine, buprenorphine, butorphanol tartrate, methadone, levomethadone, alfentanil, sufentanil, remifentanil, nalbuphine, pentazocine, oxymorphone, hydromorphone, preferably from fentanyl, morphine, oxymorphine, buprenorphine and butorphanol tartrate.

16. MK-467 for use in facilitating onset of sedation and/or anesthesia according to claim 14 or 15, **characterized in that** the benzodiazepine is selected from midazolam, zolazepam, and diazepam.

17. MK-467 for use in facilitating onset of sedation and/or anesthesia according to any one of claims 14-16, **characterized in that** the composition further comprises an NMDA receptor antagonist selected from ketamine, tiletamine, dextromethorphan, phencyclidine and methoxetamine, preferably ketamine.

18. MK-467 for use in facilitating onset of sedation and/or anesthesia according to any one of claims 14-17, **characterized in that** the α₂-adrenoceptor agonist is selected from medetomidine, dexmedetomidine, detomidine, romifidine, clonidine and xylazine.

19. MK-467 for use in facilitating onset of sedation and/or anesthesia according to any one of claims 14-18, **characterized in that** the subject is selected from humans and animals, preferably from wild animals and domestic animals.

20. MK-467 for use in facilitating onset of sedation and/or anesthesia according to claim 19, **characterized in that** the animals are selected from vertebrates, preferably from mammals, fish, birds and reptiles.

21. MK-467 for use in facilitating onset of sedation and/or anesthesia according to any one of claims 14-20, **characterized in that** the composition is administered to a subject to provide a dosage of 1-5000 µg/kg of MK-467 and 1-5000 µg/kg of the psychoactive drug opioid, benzodiazepine and NMDA receptor antagonist.

22. MK-467 for use in facilitating onset of sedation and/or anesthesia according to any one of claims 14-21, **characterized in that** the composition is administered to a subject to provide a dosage of 1-5000 µg/kg of MK-467, 1-5000 µg/kg of the psychoactive drug and 0.05-20000 µg/kg of the α₂-adrenoceptor agonist.

23. MK-467 for use in facilitating onset of sedation and/or anesthesia according to any one of claims 14-22, **characterized in that** the composition is selected from solutions for intramuscular use, intraperitoneal use and subcutaneous use; from gels, sprays, ointments, creams and patches for transdermal use; and from gels, ointments, creams, sprays and suppositories for transmucosal use.

24. MK-467 for use in facilitating onset of sedation and/or anesthesia according to any one of claims 14-23, **characterized in that** the composition is a solution for intramuscular use, intraperitoneal use or subcutaneous use and it comprises 0.1-500 mg/ml of MK-467 and 0.1-500 mg/ml of the psychoactive drug.

25. MK-467 for use in facilitating onset of sedation and/or anesthesia according to any one of claims 14-23, **characterized in that** the composition is a gel, spray, ointment, cream or patch for transdermal use and it comprises 0.1-1000 mg/ml of MK-467 and 0.1-500 mg/ml of the psychoactive drug and 0.01-100 mg/ml of the α₂-adrenoceptor agonist.

26. MK-467 for use in facilitating onset of sedation and/or anesthesia according to any one of claims 14-23, **characterized in that** the composition is a gel, ointment, cream, spray or suppository for transmucosal I use and it comprises 0.1-1000 mg/ml of MK-467 and 0.1-500 mg/ml of the psychoactive drug and 0.01 and 0.01-100 mg/ml of the α₂-adrenoceptor agonist.

## Patentansprüche

1. Zusammensetzung zur Verwendung bei einer Sedierung und/oder Anästhesie, **gekennzeichnet dadurch, dass** die Zusammensetzung eine Kombination aus MK-467, einem α₂-Adrenozeptor-Agonisten, einem Opioid und einem Benzodiazepin umfasst, wobei die Zusammensetzung einem Subjekt, das eine Sedierung und/oder Anästhesie benötigt, zur gleichen Zeit und an der gleichen Verabreichungsstelle durch parenterale extravaskuläre Verabreichung lokal verabreicht wird.

2. Zusammensetzung zur Verwendung nach Anspruch 1, **gekennzeichnet dadurch, dass** das Opioid ausgewählt ist aus Fentanyl, Morphin, Oxymorphin, Buprenorphin, Butorphanoltartrat, Methadon, Levomethadon, Alfentanil, Sufentanil, Remifentanil, Nalbuphin, Pentazocin, Oxymorphon und Hydromorphon, vorzugsweise aus Fentanyl, Morphin, Oxymorphin, Buprenorphin und Butorphanoltartrat.

3. Zusammensetzung zur Verwendung nach Anspruch 1 oder 2, **gekennzeichnet dadurch, dass** das Benzodiazepin ausgewählt ist aus Midazolam, Zolazepam und Diazepam.

4. Zusammensetzung zur Verwendung nach einem der Ansprüche 1 bis 3, **gekennzeichnet dadurch, dass** die Zusammensetzung ferner einen NMDA-Rezeptor-Antagonisten umfasst, der ausgewählt ist aus Ketamin, Tiletamin, Dextromethorphan, Phencyclidin und Methoxetamin, vorzugsweise Ketamin.

5. Zusammensetzung zur Verwendung nach einem der Ansprüche 1 bis 4, **gekennzeichnet dadurch, dass** der α₂-Adrenozeptor-Agonist ausgewählt ist aus Medetomidin, Dexmedetomidin, Detomidin, Romifidin, Clonidin und Xylazin.

6. Zusammensetzung zur Verwendung nach einem der Ansprüche 1 bis 5, **gekennzeichnet dadurch, dass** das Subjekt ausgewählt ist aus Menschen und Tieren, vorzugsweise aus Wildtieren und Haustieren.

7. Zusammensetzung zur Verwendung nach Anspruch 6, **gekennzeichnet dadurch, dass** die Tiere ausgewählt sind aus Wirbeltieren, vorzugsweise Säugetieren, Fischen, Vögeln und Reptilien.

8. Zusammensetzung zur Verwendung nach einem der Ansprüche 1 bis 7, **gekennzeichnet dadurch, dass** die Zusammensetzung einem Subjekt verabreicht wird, um eine Dosis von 1-5000 µg/kg MK-467 und 1-5000 µg/kg der psychoaktiven Substanz Opioid, Benzodiazepin und NMDA-Rezeptor-Antagonist bereitzustellen.

9. Zusammensetzung zur Verwendung nach einem der Ansprüche 1 bis 8, **gekennzeichnet dadurch, dass** die Zusammensetzung einem Subjekt verabreicht wird, um eine Dosis von 1-5000 µg/kg MK-467, 1-5000 µg/kg der psychoaktiven Substanz und 0,05-20000 µg/kg des α₂-Adrenozeptor-Agonisten bereitzustellen.

10. Zusammensetzung zur Verwendung nach einem der Ansprüche 1 bis 9, **gekennzeichnet dadurch, dass** die Zusammensetzung ausgewählt ist aus Lösungen zur intramuskulären Verwendung, intraperitonealen Verwendung und subkutanen Verwendung; aus Gelen, Sprays, Salben, Cremes und Pflastern zur transdermalen Verwendung; und aus Gelen, Salben, Cremes, Sprays und Suppositorien zur transmukosalen Verwendung.

11. Zusammensetzung zur Verwendung nach einem der Ansprüche 1 bis 10, **gekennzeichnet dadurch, dass** die Zusammensetzung eine Lösung zur intramuskulären Verwendung, intraperitonealen Verwendung oder subkutanen Verwendung ist und 0,1-500 mg/ml MK-467 und 0,1-500 mg/ml der psychoaktiven Substanz umfasst.

12. Zusammensetzung zur Verwendung nach einem der Ansprüche 1 bis 10, **gekennzeichnet dadurch, dass** die Zusammensetzung ein Gel, ein Spray, eine Salbe, eine Creme oder ein Pflaster zur transdermalen Verwendung ist und 0,1-1000 mg/ml MK-467 und 0,1-500 mg/ml der psychoaktiven Substanz und 0,01-500 mg/ml des α₂-Adrenozeptor-Agonisten umfasst.

13. Zusammensetzung zur Verwendung nach einem der Ansprüche 1 bis 10, **gekennzeichnet dadurch, dass** die Zusammensetzung ein Gel, eine Salbe, ein Spray oder ein Suppositorium zur transmukosalen Verwendung ist und 0,1-1000 mg/ml MK-467 und 0,1-500 mg/ml der psychoaktiven Substanz und 0,01-100 mg/ml des α₂-Adrenozeptor-Agonisten umfasst.

14. MK-467 zur Verwendung bei der Erleichterung des Eintretens einer Sedierung und/oder Anästhesie, **gekennzeichnet dadurch, dass** eine Zusammensetzung, die eine Kombination aus MK-467, einem α₂-Adrenozeptor-Agonisten, einem Opioid und einem Benzodiazepin umfasst, einem Subjekt, das eine Anästhesie und/oder Sedierung benötigt, zur gleichen Zeit und an der gleichen Verabreichungsstelle durch parenterale extravaskuläre Verabreichung lokal verabreicht wird.

15. MK-467 zur Verwendung bei der Erleichterung des Eintretens einer Sedierung und/oder Anästhesie nach Anspruch 14, **gekennzeichnet dadurch, dass** das Opioid ausgewählt ist aus Fentanyl, Morphin, Oxymorphin, Buprenorphin, Butorphanoltartrat, Methadon, Levomethadon, Alfentanil, Sufentanil, Remifentanil, Nalbuphin, Pentazocin, Oxymorphon, Hydromorphon, vorzugsweise aus Fentanyl, Morphin, Oxymorphin, Buprenorphin und Butorphanoltartrat.

16. MK-467 zur Verwendung bei der Erleichterung des Eintretens einer Sedierung und/oder Anästhesie nach Anspruch 14 oder 15, **gekennzeichnet dadurch, dass** das Benzodiazepin ausgewählt ist aus Midazolam, Zolazepam und Diazepam.

17. MK-467 zur Verwendung bei der Erleichterung des Eintretens einer Sedierung und/oder Anästhesie nach einem der Ansprüche 14 bis 16, **gekennzeichnet dadurch, dass** die Zusammensetzung ferner einen NMDA-Rezeptor-Antagonisten umfasst, der ausgewählt ist aus Ketamin, Tiletamin, Dextromethorphan, Phencyclidin und Methoxetamin, vorzugsweise Ketamin.

18. MK-467 zur Verwendung bei der Erleichterung des Eintretens einer Sedierung und/oder Anästhesie nach einem der Ansprüche 14 bis 17, **gekennzeichnet dadurch, dass** der α₂-Adrenozeptor-Agonist ausgewählt ist aus Medetomidin, Dexmedetomidin, Detomidin, Romifidin, Clonidin und Xylazin.

19. MK-467 zur Verwendung bei der Erleichterung des Eintretens einer Sedierung und/oder Anästhesie nach einem der Ansprüche 14 bis 18, **gekennzeichnet dadurch, dass** das Subjekt ausgewählt ist aus Menschen und Tieren, vorzugsweise aus Wildtieren und Haustieren.

20. MK-467 zur Verwendung bei der Erleichterung des Eintretens einer Sedierung und/oder Anästhesie nach Anspruch 19, **gekennzeichnet dadurch, dass** die Tiere ausgewählt sind aus Wirbeltieren, vorzugsweise Säugetieren, Fischen, Vögeln und Reptilien.

21. MK-467 zur Verwendung bei der Erleichterung des Eintretens einer Sedierung und/oder Anästhesie nach einem der Ansprüche 14 bis 20, **gekennzeichnet dadurch, dass** die Zusammensetzung einem Subjekt verabreicht wird, um eine Dosis von 1-5000 µg/kg MK-467 und 1-5000 µg/kg der psychoaktiven Substanz Opioid, Benzodiazepin und NMDA-Rezeptor-Antagonist bereitzustellen.

22. MK-467 zur Verwendung bei der Erleichterung des Eintretens einer Sedierung und/oder Anästhesie nach einem der Ansprüche 14 bis 21, **gekennzeichnet dadurch, dass** die Zusammensetzung einem Subjekt verabreicht wird, um eine Dosis von 1-5000 µg/kg MK-467, 1-5000 µg/kg der psychoaktiven Substanz und 0,05-20000 µg/kg des α₂-Adrenozeptor-Agonisten bereitzustellen.

23. MK-467 zur Verwendung bei der Erleichterung des Eintretens einer Sedierung und/oder Anästhesie nach einem der Ansprüche 14 bis 22, **gekennzeichnet dadurch, dass** die Zusammensetzung ausgewählt ist aus Lösungen zur intramuskulären Verwendung, intraperitonealen Verwendung und subkutanen Verwendung; aus Gelen, Sprays, Salben, Cremes und Pflastern zur transdermalen Verwendung; und aus Gelen, Salben, Cremes, Sprays und Suppositorien zur transmukosalen Verwendung.

24. MK-467 zur Verwendung bei der Erleichterung des Eintretens einer Sedierung und/oder Anästhesie nach einem der Ansprüche 14 bis 23, **gekennzeichnet dadurch, dass** die Zusammensetzung eine Lösung zur intramuskulären Verwendung, intraperitonealen Verwendung oder subkutanen Verwendung ist und 0,1-500 mg/ml MK-467 und 0,1-500 mg/ml der psychoaktiven Substanz umfasst.

25. MK-467 zur Verwendung bei der Erleichterung des Eintretens einer Sedierung und/oder Anästhesie nach einem der Ansprüche 14 bis 23, **gekennzeichnet dadurch, dass** die Zusammensetzung ein Gel, ein Spray, eine Salbe, eine Creme oder ein Pflaster zur transdermalen Verwendung ist und 0,1-1000 mg/ml MK-467 und 0,1-500 mg/ml der psychoaktiven Substanz und 0,01-100 mg/ml des α₂-Adrenozeptor-Agonisten umfasst.

26. MK-467 zur Verwendung bei der Erleichterung des Eintretens einer Sedierung und/oder Anästhesie nach einem der Ansprüche 14 bis 23, **gekennzeichnet dadurch, dass** die Zusammensetzung ein Gel, eine Salbe, ein Spray oder ein Suppositorium zur transmukosalen Verwendung ist und 0,1-1000 mg/ml MK-467 und 0,1-500 mg/ml der psychoaktiven Substanz und 0,01-100 mg/ml des α₂-Adrenozeptor-Agonisten umfasst.

## Revendications

1. Composition pour une utilisation en sédation et/ou en anesthésie, **caractérisée en ce que** la composition comprend une combinaison de MK-467, d'un agoniste des récepteurs α₂-adrénergiques, d'un opioïde et d'une benzodiazépine, la composition étant administrée localement par voie parentérale extravasculaire, au même moment et au même site d'administration, à un sujet nécessitant une sédation et/ou une anesthésie.

2. Composition pour l'utilisation selon la revendication 1, **caractérisée en ce que** l'opioïde est choisi parmi le fentanyl, la morphine, l'oxymorphine, la buprénorphine, le tartrate de butorphanol, la méthadone, la lévométhadone, l'alfentanil, le sufentanil, le rémifentanil, la nalbuphine, la pentazocine, l'oxymorphone et l'hydromorphone, de préférence parmi le fentanyl, la morphine, l'oxymorphine, la buprénorphine et le tartrate de butorphanol.

3. Composition pour l'utilisation selon la revendication 1 ou 2, **caractérisée en ce que** la benzodiazépine est choisie parmi le midazolam, le zolazépam et le diazépam.

4. Composition pour l'utilisation selon l'une des revendications 1 à 3, **caractérisé en ce que** la composition comprend également un antagoniste des récepteurs NMDA choisi parmi la kétamine, la tilétamine, le dextrométhorphane, la phencyclidine et la méthoxétamine, de préférence la kétamine.

5. Composition pour l'utilisation selon l'une des revendications 1 à 4, **caractérisée en ce que** l'agoniste des récepteurs α₂-adrénergiques est choisi parmi la médétomidine, la dexmédétomidine, la détomidine, la romifidine, la clonidine et la xylazine.

6. Composition pour l'utilisation selon l'une des revendications 1 à 5, **caractérisée en ce que** le sujet est choisi parmi les humains et les animaux, de préférence les animaux sauvages et les animaux domestiques.

7. Composition pour l'utilisation selon la revendication 6, **caractérisée en ce que** les animaux sont choisis parmi les vertébrés, de préférence parmi les mammifères, les poissons, les oiseaux et les reptiles.

8. Composition pour l'utilisation selon l'une des revendications 1 à 7, **caractérisée en ce que** la composition est administrée à un sujet pour fournir une dose de 1-5000 µg/kg de MK-467 et de 1-5000 µg/kg de la substance psychoactive opioïde, benzodiazépine et antagoniste des récepteurs NMDA.

9. Composition pour l'utilisation selon l'une des revendications 1 à 8, **caractérisée en ce que** la composition est administrée à un sujet pour fournir une dose de 1-5000 µg/kg de MK-467, de 1-5000 µg/kg de la substance psychoactive et de 0,05-20000 µg/kg de l'agoniste des récepteurs α2-adrénergiques.

10. Composition pour l'utilisation selon l'une des revendications 1 à 9, **caractérisée en ce que** la composition est choisie parmi les solutions pour une utilisation intramusculaire, une utilisation intrapéritonéale et une utilisation sous-cutanée; parmi les gels, sprays, pommades, crèmes et patchs pour une utilisation transdermique; et parmi les gels, pommades, crèmes, sprays et suppositoires pour une utilisation transmucosale.

11. Composition pour l'utilisation selon l'une des revendications 1 à 10, **caractérisée en ce que** la composition est une solution pour une utilisation intramusculaire, une utilisation intrapéritonéale ou une utilisation sous-cutanée et elle comprend 0,1-500 mg/ml de MK-467 et 0,1-500 mg/ml de la substance psychoactive.

12. Composition pour l'utilisation selon l'une des revendications 1 à 10, **caractérisée en ce que** la composition est un gel, un spray, une pommade, une crème ou un patch pour une utilisation transdermique et elle comprend 0,1-1000 mg/ml de MK-467 et 0,1-500 mg/ml de la substance psychoactive et 0,01-500 mg/ml de l'agoniste des récepteurs α₂-adrénergiques.

13. Composition pour l'utilisation selon l'une des revendications 1 à 10, **caractérisée en ce que** la composition est un gel, une pommade, une crème, un spray ou un suppositoire pour une utilisation transmucosale et elle comprend 0,1-1000 mg/ml de MK-467 et 0,1-500 mg/ml de la substance psychoactive et 0,01-100 mg/ml de l'agoniste des récepteurs α₂-adrénergiques.

14. MK-467 pour une utilisation visant à faciliter l'apparition de la sédation et/ou de l'anesthésie, **caractérisé en ce que** la composition comprenant une combinaison de MK-467, d'un agoniste des récepteurs α₂-adrénergiques, d'un opioïde et d'une benzodiazépine est administrée localement par voie parentérale extravasculaire, au même moment et au même site d'administration, à un sujet nécessitant une anesthésie et/ou une sédation.

15. MK-467 pour l'utilisation visant à faciliter l'apparition de la sédation et/ou de l'anesthésie selon la revendication 14, **caractérisé en ce que** l'opioïde est choisi parmi le fentanyl, la morphine, l'oxymorphine, la buprénorphine, le tartrate de butorphanol, la méthadone, la lévométhadone, l'alfentanil, le sufentanil, le rémifentanil, la nalbuphine, la pentazocine, l'oxymorphone, l'hydromorphone, de préférence parmi le fentanyl, la morphine, l'oxymorphine, la buprénorphine et le tartrate de butorphanol.

16. MK-467 pour l'utilisation visant à faciliter l'apparition de la sédation et/ou de l'anesthésie selon la revendication 14 ou 15, **caractérisé en ce que** la benzodiazépine est choisie parmi le midazolam, le zolazépam et le diazépam.

17. MK-467 pour l'utilisation visant à faciliter l'apparition de la sédation et/ou de l'anesthésie selon l'une des revendications 14 à 16, **caractérisé en ce que** la composition comprend également un antagoniste des récepteurs NMDA choisi parmi la kétamine, la tilétamine, le dextrométhorphane, la phencyclidine et la méthoxétamine, de préférence la kétamine.

18. MK-467 pour l'utilisation visant à faciliter l'apparition de la sédation et/ou de l'anesthésie selon l'une des revendications 14 à 17, **caractérisé en ce que** l'agoniste des récepteurs α₂-adrénergiques est choisi parmi la médétomidine, la dexmédétomidine, la détomidine, la romifidine, la clonidine et la xylazine.

19. MK-467 pour l'utilisation visant à faciliter l'apparition de la sédation et/ou de l'anesthésie selon l'une des revendications 14 à 18, **caractérisé en ce que** le sujet est choisi parmi les humains et les animaux, de préférence les animaux sauvages et les animaux domestiques.

20. MK-467 pour l'utilisation visant à faciliter l'apparition de la sédation et/ou de l'anesthésie selon la revendication 19, **caractérisé en ce que** les animaux sont choisis parmi les vertébrés, de préférence parmi les mammifères, les poissons, les oiseaux et les reptiles.

21. MK-467 pour l'utilisation visant à faciliter l'apparition de la sédation et/ou de l'anesthésie selon l'une des revendications 14 à 20, **caractérisé en ce que** la composition est administrée à un sujet pour fournir une dose de 1-5000 µg/kg de MK-467 et de 1-5000 µg/kg de la substance psychoactive opioïde, benzodiazépine et antagoniste des récepteurs NMDA.

22. MK-467 pour l'utilisation visant à faciliter l'apparition de la sédation et/ou de l'anesthésie selon l'une des revendications 14 à 21, **caractérisé en ce que** la composition est administrée à un sujet pour fournir une dose de 1-5000 µg/kg de MK-467, de 1-5000 µg/kg de la substance psychoactive et de 0,05-20000 µg/kg de l'agoniste des récepteurs α2-adrénergiques.

23. MK-467 pour l'utilisation visant à faciliter l'apparition de la sédation et/ou de l'anesthésie selon l'une des revendications 14 à 22, **caractérisé en ce que** la composition est choisie parmi les solutions pour une utilisation intramusculaire, une utilisation intrapéritonéale et une utilisation sous-cutanée; parmi les gels, sprays, pommades, crèmes et patchs pour une utilisation transdermique; et parmi les gels, pommades, crèmes, sprays et suppositoires pour une utilisation transmucosale.

24. MK-467 pour l'utilisation visant à faciliter l'apparition de la sédation et/ou de l'anesthésie selon l'une des revendications 14 à 23, **caractérisé en ce que** la composition est une solution pour une utilisation intramusculaire, une utilisation intrapéritonéale ou une utilisation sous-cutanée et elle comprend 0,1-500 mg/ml de MK-467 et 0,1-500 mg/ml de la substance psychoactive.

25. MK-467 pour l'utilisation visant à faciliter l'apparition de la sédation et/ou de l'anesthésie selon l'une des revendications 14 à 23, **caractérisé en ce que** la composition est un gel, un spray, une pommade, une crème ou un patch pour une utilisation transdermique et elle comprend 0,1-1000 mg/ml de MK-467 et 0,1-500 mg/ml de la substance psychoactive et 0,01-100 mg/ml de l'agoniste des récepteurs α₂-adrénergiques.

26. MK-467 pour l'utilisation visant à faciliter l'apparition de la sédation et/ou de l'anesthésie selon l'une des revendications 14 à 23, **caractérisé en ce que** la composition est un gel, une pommade, une crème, un spray ou un suppositoire pour une utilisation transmucosale et elle comprend 0,1-1000 mg/ml de MK-467 et 0,1-500 mg/ml de la substance psychoactive et 0,01-100 mg/ml de l'agoniste des récepteurs α₂-adrénergiques.
